# EUROPEAN PATENT SPECIFICATION

(11) **EP 2 729 003 B1**
(45) Date of publication and mention of the grant of the patent: **20.06.2018**
(21) Application number: 12761647.2
(22) Date of filing: 04.07.2012
(51) Int. Cl.: A01N 33/12, A01N 33/24, A01N 47/44, A61L 2/235

(54) **BIOCIDAL COMPOSITION COMPRISING A BIGUANIDE POLYMER, A QUATERNARY AMMONIUM SALT AND A TERTIARY AMINE OXIDE**
BIOZIDE ZUBEREITUNGEN ENTHALTEND EIN BIGUANID POLYMER, EIN QUATERNÄRES AMMONIUMSALZ UND EIN TERTIÄRES AMINOXID
COMPOSITION BIOCIDE COMPRENANT UN POLYMER DE BIGUANIDE, UN SEL D'AMMONIUM QUATERNAIRE ET UN OXYDE D'AMINE TERTIAIRE

(30) Priority: 05.07.2011 GB 201111490
(43) Date of publication of application: 14.05.2014
(73) Proprietor: Bio Technics Limited, Gourdon, Aberdeenshire DD10 0NH (GB)
(72) Inventor: EVANS, David Thomas, Gourdon Aberdeenshire DD10 0NH (GB); HOWE, Timothy Mark, Dundee DD10 0SL (GB)
(74) Representative: Hindles Limited
(86) International application number: PCT/GB2012/051566
(87) International publication number: WO 2013/005036

(56) References cited:
- EP-A1- 1 146 112
- EP-A2- 0 265 202
- WO-A1-2010/042459
- WO-A2-2010/097639
- US-A- 4 587 266
- US-A1- 2007 258 996
- US-A1- 2010 104 528
- FRAUD ET AL.: JOURNAL OF ANTIMICROBIAL CHEMOTHERAPY, vol. 56, 2005, pages 672-677, XP55044123, cited in the application
- DATABASE WPI Week 199035 Thomson Scientific, London, GB; AN 1990-264444 & JP H02 184609 A (SANYO CHEM IND LTD) 19 July 1990 (1990-07-19)
- DATABASE WPI Week 201109 Thomson Scientific, London, GB; AN 2011-A59959 & JP 4 611445 B1 (TERAMOTO CORP LTD) 12 January 2011 (2011-01-12)
- DATABASE WPI Week 200356 Thomson Scientific, London, GB; AN 2003-595254 & RU 2 207 154 C1 (MK VITA PUL CO LTD) 27 June 2003 (2003-06-27)

## Description

### Field of the invention

The invention relates to a biocidal composition which demonstrates enhanced rate and efficacy, in particular against spore-forming bacteria such as *Clostridium Difficile.*

### Background to the invention

Nosocomial infections have always been a major issue in hospitals, nursing homes or similar institutions where residents are at an elevated risk of infection due to their failing health, their compromised immune system etc.

Antibiotics are used to kill or inhibit the growth of infectious organisms such as pathogenic bacteria. Unfortunately, the increased use of antibiotics has been accompanied by an alarming spread of antibiotic-resistant or opportunistic bacteria. *Clostridium difficile,* for example, are now a common cause of infections in hospitals. Clostridia are motile bacteria that are ubiquitous in nature and are especially prevalent in soil.

When stressed, certain bacteria and fungi produce spores, which tolerate extreme conditions that the active bacteria cannot tolerate. For example, *C. difficile* is a strain which is present in the human intestine in 2-5% of the population. In small numbers, *C. difficile* does not result in significant disease. However antibiotics, especially those with a broad spectrum of activity, cause disruption of normal intestinal flora, leading to an overgrowth of *C*. *difficile,* which flourishes under these conditions. This can lead to various inflammatory diseases.

Antibiotic treatment of *C*. *difficile* infections can be difficult, due both to antibiotic resistance as well as physiological factors of the bacteria itself (spore formation, protective effects of the pseudomembrane). As the organism forms spores which are heat resistant and not destroyed by conventional biocidal agents, *C. difficile* spores can survive normal hospital cleaning procedures and can remain in the hospital or nursing home environment for long periods of time, able to germinate when conditions are favourable, resulting in *C. difficile* infection even after deep cleaning. Furthermore, *C. difficile* can be cultured from almost any surface in the hospital. Additionally, the spores themselves may be picked up from surfaces and ingested or transferred to other surfaces. Once spores are ingested, they pass through the stomach unscathed because of their acid-resistance. They change to their active form in the colon and multiply. Pseudomembranous colitis caused by *C. difficile* is treated with specific antibiotics, for example, vancomycin, metronidazole, bacitracin or fusidic acid.

It is recognised that disinfectants can have different levels of effectiveness. In general, a disinfectant with a low level of effectiveness will be effective against some vegetative bacteria, lipid viruses and fungi, a disinfectant with an intermediate level of effectiveness will be effective against some non-lipid viruses and mycobacteria such as those causing tuberculosis, and a disinfectant with a high level of effectiveness will be effective against bacterial spores. Several disinfectants commonly used in hospitals may be ineffective against organisms which form spores, such as *C. difficile,* and may in some cases actually promote spore formation. There is therefore a strong need for the development of disinfectant compositions which exhibit efficacy against spore forming organisms such as *C*. *difficile.*

Effective sporicidal agents are known. However, conventional sporicidal agents such as hydrogen peroxide, formaldehyde and peracetic acid are highly toxic and thus not suitable for frequent use. Similarly, biocidal agents effective against other types of organism are known, which suffer from high toxicity, thus limiting the range of applications for which they are suitable.

In addition to the level of biocidal activity which a composition demonstrates (e.g. as measured in standard suspension tests), the rate at which organisms are killed is of particular importance in some applications. For example, in hand washing and surface cleaning applications it is known that the effectiveness of biocidal treatment can be compromised by a user's tendency to remove the composition before sufficient disinfection has occurred (e.g. by wiping or drying the surface, rinsing or drying hands etc.). Furthermore, the rate at which some types of organisms (e.g. bacteria) are killed by a composition is higher than for other types of organism (e.g. fungi, spores). Consequently, the overall contamination levels of some types of organisms on surfaces can build up over time, by repeatedly removing the active compositions from the surface prematurely, during cleaning.

Accordingly, there has conventionally been a trade off between increasing the concentration of the active agents present in a composition, so as to increase the rate of disinfection, versus the need to limit such concentrations so as to minimise toxicity, skin irritation, allergic reactions and environmental impact, etc.

Thus, there remains a need for more effective, rapidly acting biocidal compositions which also contain biocidally active constituents at a minimum of concentration.

US 4,587,266 discloses antimicrobial compositions comprising a bis-biguanide compound and an amidoamine oxide.

US 2010/0104528 discloses an aqueous contact lens solution comprising less than 100 ppm of a tertiary amine oxide of general formula R₁R₂R₃NO wherein R₁ is a C₈ - C₁₈ alkyl, and R₂ and R₃ are independently selected from a C₁ - C₄ alkyl or a C₁ - C₄ hydroxyalkyl, and an antimicrobial component selected from the group consisting of biguanides, polymeric biguanides, quaternium ammonium compounds, hydrogen peroxide or a stabilized form of peroxide and any one mixture thereof. The solution has no anionic surfactant or less than 30 ppm of anionic surfactant. This document is also directed to the use of the solution to clean and disinfect contact lenses, and in particular, soft, silicone, hydrogel contact lenses.

WO 2010/042459 discloses a topical pharmaceutical formulation comprising 0.5 ppm to 1000 ppm of a tertiary amine oxide. The tertiary amine oxide functions as a preservative or enhances the preservative efficacy in such formulations developed for topical administration. In particular, the topical formulation is an ophthalmic composition.

US 2007/0258996 discloses an antimicrobial composition, including a synergistic combination of three or more potentiating agents as an active ingredient. Each of the three or more potentiating agents may be selected from the following types of compounds: sequestering agents, carbohydrates, terpenes, terpenoids, peptides, alkaloids, plant-derived oils, dyes and stains, sulfonates, phenols, esters, fatty acids and dibenzofuran derivatives. At least two of the three or more potentiating agents are not of the same type of compound.

EP 0265202 A2 discloses a sanitising composition particularly suited to the cleaning-in-place of food industry equipment, comprising (i) one or more quaternary ammonium anti-microbial agents and/or one or more substituted guanidine anti-microbial agents: (ii) one or more organic acids; and, (ii) one or more inorganic acids.

EP 1146112 A1 discloses disinfecting and/or cleaning a hard-surface with a composition comprising a biguanide antimicrobial agent, an essential oil or an active thereof and acid or a salt thereof.

JP HO 2184609 A discloses a germicidal composition comprising a di C₈ - C₁₀ alkyldimethylammonium salt and a biguanide compound and further containing a non-ionic surfactant. The composition is used for sterilizing and cleaning floors or walls. In an example 15 wt.% of didecyldimethylammonium chloride, 5 w.% of polyhexamethylebiganidin chloride, 5 w.% of lauryldimethylamineoxide, and 75 w. % of water were mixed to obtain a germicidal composition.

JP 4611445 B discloses compositions for cleaning medical instruments, toilets and other items. The compositions comprise dimethyloctadecyl[3-(trimethoxysilyl)propyl]ammonium chloride and/or dimethyloctadecyl[3-(triethoxysilyl)propyl]ammonium chloride, amidoamine oxide as surfactant, polyhexamethylene biguanide compound, alcohol and water.

RU 2207154 discloses detergents used for disinfection and presterilizing cleansing medicinal articles and tools, and in particular compositions comprising polyhexamethyleneguanidine or polyhexamethylenebiguanide hydrochloride, alkyldimethyl ammonium chloride or N,N-didecyl-N,N-dimethyl ammonium chloride; N-lauryl-N,N-dimethyl amino-oxide or (C₆-C₁₆)-alkyldimethylamino-oxide; surface-active substances, dye, perfume and water.

### Summary of the invention

According to a first aspect of the invention there is provided a non-therapeutic method of killing spores on a surface which comprises applying an effective amount of a composition to said surface, wherein said composition comprises at least one biguanide polymer, at least one further active agent and at least one tertiary amine oxide, wherein the at least one further active agent comprises a quaternary ammonium salt.

We have found that the inclusion of a tertiary amine oxide in a composition comprising at least one biguanide polymer and at least one further active agent results in an unexpected enhancement of the biocidal activity of the composition, in comparison to compositions lacking a tertiary amine oxide. No synergistic effect on the biocidal activity of a biocidal composition resulting from the combination of amine oxides with any other biocides has been previously observed, in particular of the biocidal activity of compositions comprising biguanide polymers in combination with a further active agent.

Although not wishing to be bound by theory, it may be postulated that the tertiary amine oxide acts as an activity promoter by interacting with the walls of spores (including bacterial endospores) and cells (including bacterial and fungal cells, microbial cysts and the cell walls of other microbes) so as to increase their permeability to the polymeric biguanides and other active agents. Certain amine oxides are known to have antibacterial properties (for example, as disclosed in Fraud et al. Journal of Antimicrobial Chemotherapy (2005) 56, 672-677), however amine oxides have not previously been associated with particularly high levels of such activity (or, for example, fungicidal or sporicidal activity) and no synergistic relationship between amine oxides and other active agents (such as biguanide polymers or said further active agents) has been observed. Therefore the pronounced enhancement of biocidal activity that we have observed (in comparison to similar compositions lacking amine oxides) is particularly unexpected.

By active agent, and related terms, we mean active against organisms including (but not limited to) one or more of spores, including spores of bacteria, fungi, molds, algae, plants or other organisms including (but not limited to) one or more of bacteria, molds, microbes, germs, yeast, algae, viruses.

The biocidal composition may comprise a tertiary alkyl amine oxide, i.e. a tertiary amine oxide substituted with at least one alkyl group. In one embodiment, the tertiary alkyl amine oxide is substituted with three alkyl groups.

The biocidal composition may comprise a tertiary amine oxide having the general formula:

R₁R₂R₃NO

where R₁ and/or R₂ is a methyl group and R₂ and/or R₃ is a C₈-C₂₂, preferably a C₁₀-C₁₈, more preferably a C₁₀-C₁₆ and most preferably a C₁₂-C₁₄ hydrocarbon group. R₃ may be a branched or straight chain hydrocarbon group, and may be saturated or may comprise one or more unsaturated carbon-carbon bonds, and may comprise one or more substituents, such as oxygen containing substituents, (e.g. hydroxyl, carboxyl, carbonyl, ether or ester) or nitrogen containing substituents (e.g. amide or amine), or cyclic or heterocyclic substituents, or halogenated substituents (e.g. chloride, bromide or halogenated alkyl substituents). In other words, in one embodiment, R₁ and R₂ are each a methyl group and R₃ is a C₈-C₂₂, C₁₀-C₁₈, C₁₀-C₁₆, or C₁₂-C₁₄ hydrocarbon group as described above and in another embodiment, R₁ is a methyl group and R₂ and R₃ are each a C₈-C₂₂, C₁₀-C₁₈, C₁₀-C₁₆ or C₁₂-C₁₄ hydrocarbon group as described above.

Typically, R₂ or R₃ is an alkyl group and one or more of the or each said tertiary amine oxide may, for example, be lauryldimethylamine oxide (N,N-dimethyldodecan-1-amine oxide, CAS No. 1643-20-5).

The biocidal composition may comprise a mixture of tertiary amine oxides. The R₂ or R₃ of the mixture of tertiary amine oxides may comprise a range of hydrocarbon chain lengths such as C10-C16, for example.

Advantageously, tertiary alkyl amine oxides and in particular those comprising long chain (e.g. C₈-C₂₂) alkyl or other groups are both water soluble (and are thus suitable for use in aqueous and alcohol free compositions) and possess surfactant properties, and thus additionally improve the interaction of the composition with surfaces.

Whereas it is known that other components may be added to a biguanide containing disinfectant composition in order to promote biocidal activity, in particular sequestering and chelating agents (e.g. ethylenediaminetetraacetic acid (EDTA), ethylenediamine-N,N'-disuccinic acid (EDDS), nitrilotriacetic acid (NTA), succinates, cryptands, crown ethers, organic acids, organophosphates), such species are not effective surfactants, such that further surfactants are typically required, particularly in order to produce a foamable formulation. Furthermore, the enhancement of activity associated with these species, i.e. in relation to compositions lacking an activity enhancing sequestering/chelating agent, has not been observed to be as pronounced as has been observed in relation to amine oxides.

Preferably, the at least one said biguanide polymer, the at least one further active agent and the at least one said tertiary amine oxide are, when taken alone, sporicidally inactive.

By the sporicidal or biocidal activity of an active agent, when taken alone, we mean the activity of the active agent in biocidal compositions lacking another biocidal agent. Sporicidal activity or inactivity is assessed in relation to the minimum requirements to pass standard suspension test EN 13704:2002 in clean or dirty conditions, in relation to a log₁₀3 reduction in viable spore concentration in a period of 60 minutes. The said test is generally carried out against *Bacillus subtilis*, *Bacillus cereus* or *Clostridium sporogenes.*

The sporicidal or biocidal activity of an active agent may be assessed in a modified suspension test whereby the standard protocol is followed but the active agent is tested against *Clostridium difficile* rather than those discussed above. Fast acting agents may be assessed in a further modified suspension test where the period of the test is modified to a period of 30 minutes, a period of 10 minutes or preferably a period of 5 minutes.

Preferably the one or more further active agents are sporicidally inactive at much greater concentrations (such as ten times, or greater than ten times the concentration of the active agent in non-toxic compositions as required for the EN 13704 standard test), or at any concentration. The one or more further active agents may be sporicidally inactive in their pure form.

By sporicide and sporicidally active, and related terms, we mean active against spores, including spores of bacteria, fungi, molds, algae, plants or other organisms, including in particular bacterial endospores. Accordingly, a sporicidally active agent may be active against spores including endospores. Additionally, an active agent which is, when taken alone, sporicidally inactive may be endosporicidally inactive (for example inactive against bacterial endospores) but may (or may not) be active against other types of spores.

The unexpected sporicidal activity of compositions comprising biocidally active agents which are not, when taken alone, sporicidally active, has been previously observed (WO 2010/097639). However, previously known compositions have typically been shown to be capable of providing moderate and in some cases long-lasting sporicidal activity to a surface, the rate at which spores are killed has been comparatively slow (for example a log₁₀3 reduction in viable spore concentrations over a period of 10 or more minutes), limiting the effectiveness of such formulations in certain applications (for example manual cleaning of surfaces or hands). In contrast, compositions of the present invention, comprising biguanide polymer, one or more further active agents and one or more amine oxides, each of which are not sporicidally active, when taken alone, exhibit a significantly greater log reduction in viable spore concentrations, achieved in a significantly reduced time, in comparison to previously known compositions.

Preferably, the composition is a foaming or foamable composition, i.e. the composition is preferably dispensable as a foam, or may be caused to form a foam by appropriate agitation. (By "foam" we mean a structure of polydisperse gas bubbles (preferably air bubbles) separated by films of the composition, which is substantially stable for a period of the order of at least 10 seconds.)

A formulation applied to a surface to be disinfected as a foam does not become airborne (and so is less prone to inhalation than a composition applied as a spray or aerosol) and is less prone to running or dripping from surfaces which are not horizontal, in comparison to compositions applied as a liquid (sprayed, or otherwise).

Certain long chain tertiary amine oxides (e.g. comprising one or more alky groups having C₈-C₂₂ chains, such as lauryldimethyamine oxide) are known to have surfactant properties. Therefore, advantageously, compositions comprising certain long chain tertiary amine oxides are foaming or foamable (and typically aqueous) compositions and are provided with both their foaming or foamable properties, and their enhanced biocidal activity, by the presence of the said amine oxide. Accordingly, the need for additional surfactant or biocidal agents can be reduced or avoided.

The composition comprises a quaternary ammonium salt as further active agent.

The composition may comprise at least two further active agents. Some amine oxides are, when taken alone, known to be biocidally active. Thus, in some embodiments, the composition may comprise at least two further active agents, of which at least one is a said tertiary amine oxide.

More further active agents may be selected from the group consisting of a biocidal, anti-microbial, bactericidal, fungicidal, germicidal, yeasticidal, moldicidal, algicidal, virucidal agent, or a mixture thereof.

Further active agents may be selected from cationic, amphoteric, amino, phenolic and halogen containing biocides. Examples of the biocides which may be used include:
Cationic biocides such as:
- quaternary monoammonium salts, for example cocoalkylbenzyldimethylammonium chloride, C8-C18 alkylbenzyldimethylammonium chloride, cocoalkyldichlorobenzyldimethylammonium chloride, tetradecylbenzyldimethylammonium chloride, di-n-decyldimethylammonium chloride and dioctyldimethylammonium chloride,
- Myristyltrimethylammonium bromide and cetyltrimethylammonium bromide,
- Monoquaternary heterocyclic amine salts such as laurylpyridinium, cetylpyridinium chloride and C12 - C14 alkylbenzyldimethylammonium chloride,
- Triphenylphosphonium fatty alkyl salts such as myristyltriphenyiphosphonium bromide;

Polymeric biocides such as those derived from the reactions:
- Of epichlorohydrin and dimethylamine or diethylamine,
- Of epichlorohydrin and imidazole,
- Of 1,3-dichloro-2-propanol and dimethylamine,
- Of 1,3-dichloro-2-propanol and 1,3-bis (dimethylamino)-2 propanol,
- Of ethylene dichloride and 1,3-bis(dimethylamino)-2-propanol, and
- Of bis (2-chloroethyl) ether and of N, N'-bis (dimethylaminopropyl)urea or thiourea.

Amphoteric biocides such as derivatives of N-(N'-C8-C18 alkyl1-3-aminopropyl)glycine, of N-(N'-(N"-C8-C18 alky1-2-aminoethyl)-2-aminoethyl) glycine, of N,N-bis(N'-C8-C18 alkyl-2-aminoethyl)glycine, such as (dodecyl) (aminopropyl) glycine and (dodecyl) (diethylenediamine) glycine;
Amines such as N-(3-aminopropyl)-N-dodecyl-1,3-propanediamine;
Phenolic biocides such as para-chloro-meta-xylenol, dichloro-meta-xylenol, phenol, cresols, resorcinol, resorcinol monoacetate, and their derivatives or water-soluble salts;
Halogenated biocides such as iodophores and hypochlorite salts, for example sodium dichloroisocyanurate;
5-chloro-2-methyl-4-isothiazolin-3-one; 1,2 benzisothiazol-3(2H)-one; 2-methyl-isothiazol-3(2H)- one; and 2-methyl-4-isothiazolin-3-one.

Thus the composition of the invention can comprise more active agents selected from: quaternary monoammonium salts, triphenylphosphonium fatty alkyl salts, a polymeric biocide obtainable from the reaction of epichlorohydrin and dimethylamine or diethylamine, a polymeric biocide obtainable from the reaction of epichlorohydrin and imidazole, a polymeric biocide obtainable from the reaction of 1,3-dichloro-2-propanol and dimethylamine, a polymeric biocide obtainable from the reaction of 1,3-dichloro-2-propanol and 1,3-bis(dimethylamino)-2-propanol, a polymeric biocide obtainable from the reaction of ethylene dichloride and 1,3-bis(dimethylamino)-2-propanol, a polymeric biocide derivable from the reaction of bis(2-chloroethyl)ether and N,N'-bis(dimethylaminopropyl)urea or thiourea, derivatives of N-(N'-C8-C18 alky1-3-aminopropyl)glycine, derivatives of N-(N'-(N"-C8-C18 alkyl1-2-aminoethyl)-2-aminoethyl) glycine, derivatives of N,N-bis(N'-C8-C18 alkyl-2-aminoethyl)glycine, amines, para-chloro-meta-xylenol and its derivatives or water-soluble salts, dichloro-metaxylenol and its derivatives or water-soluble salts, phenol and its derivatives or water-soluble salts, cresols and their derivatives or water-soluble salts, resorcinol and its derivatives or water-soluble salts, resorcinol monoacetate, iodophores, hypochlorite salts, 5-chloro-2-methyl-4-isothiazolin-3-one, 1,2 benzisothiazol-3(2H)-one and 2-methyl-4-isothiazolin-3-one.

In some embodiments, the formulation comprises at least one biguanide polymer and at least two quaternary ammonium salts. Thus, in one embodiment, the formulation comprises two or more quaternary ammonium salts. Preferably, at least one of the quaternary ammonium salts is a benzyl-substituted quaternary ammonium salt. More preferably the formulation comprises at least one alkyl-substituted quaternary ammonium salt and at least one benzyl-substituted quaternary ammonium salt.

By alkyl-substituted quaternary ammonium salt is meant a quaternary ammonium salt substituted by at least one alkyl group. Preferably the at least one alkyl-substituted quaternary ammonium salt is not substituted by a benzyl group. The alkyl-substituted quaternary ammonium salt can be represented by the following formula:

NR₄R₅RₑR₇⁺X⁻

wherein each of R₄ to R₇ is an alkyl group. Preferably, each of R₄ to R₇ is an alkyl group chosen from C₁ to C₁₈ alkyl groups. In one embodiment, each of R₄ and R₅ are methyl groups and R₆ and R₇ are each chosen from C₇ to C₁₈, or, preferably, C₈-C₁₀, alkyl groups. In one embodiment, each of R₄ and R₅ are methyl groups and: R₆ and R₇ are each a C₈ alkyl group; or R₆ and R₇ are each a C₁₀ alkyl group; or R₆ is a C₈ alkyl group and R₇ is a C₁₀ alkyl group. X is a counter ion such as a halide, e.g. X can be a chloride, bromide or iodide ion. Examples of alkyl-substituted quaternary ammonium salts are di-n-decyl dimethyl ammonium chloride, dioctyldimethyl -ammonium chloride and octyldecyldimethylammonium chloride.

By benzyl-substituted quaternary ammonium salt is meant a quaternary ammonium salt substituted by at least one benzyl group. The benzyl-substituted quaternary ammonium salt can be represented by the following formula:

NR₈R₉R₁₀R₁₁⁺X⁻

wherein R₈ to R₁₁ are chosen from alkyl groups and a benzyl group wherein at least one of R₈ to R₁₁ is a benzyl group. The alkyl groups are preferably chosen from C₁ to C₁₈ alkyl groups. The benzyl group may be unsubstituted or substituted with, for example, one or more halogen atoms, for example chlorine, iodine or bromine atoms. In one embodiment, R₈ and R₉ are each methyl groups, R₁₀ is chosen from C₇ to C₁₈, alkyl groups and R₁₁ is a benzyl group. In one embodiment, R₈ and R₉ are each methyl groups, R₁₀ is chosen from C₈ to C₁₁, C₁₂ to C₁₆ or C₁₂ to C₁₄ alkyl groups and R₁₁ is a benzyl group. R₁₁ can be a dichlorobenzyl group. X is a counter ion such as a halide, e.g. X can be a chloride, iodide or bromide ion. Examples of the benzyl-substituted quaternary ammonium salt are cocoalkylbenzyldimethylammonium chloride, C₈-C₁₈ or C₁₂-C₁₄ alkylbenzyldimethylammonium chloride, cocoalkyldichloro -benzyldimethylammonium chloride, tetradecylbenzyldimethylammonium chloride.

In one embodiment, the formulation comprises at least one biguanide polymer and at least two quaternary ammonium salts as described above and said formulation does not comprise an isothiazolone or an isothiazolinone compound. The formulation of this embodiment has an advantage over isothiazolone/isothiazolinone-containing formulations in that it is safer to use in spray applications and in applications involving skin-contact. In particular, in this embodiment is suitable for use as a hand sanitizer.

The biguanide polymer is preferably a biguanide polymer hydrochloride.

Biguanide polymers are formed from monomers containing the functional group: 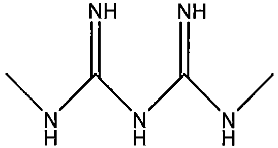 and may be separated by carbon chains of any length. The composition typically comprises straight chain C₄-C₈ biguanides, for example, polyaminopropyl biguanide (PAPB; a C₅ biguanide polymer) or polyhexamethylene biguanide (PHMB; a C₆ biguanide polymer). PHMB is a polymer which comprises biguanide functional groups which are connected by hexyl hydrocarbon chains, with varying chain lengths. It can be represented by the general structure:

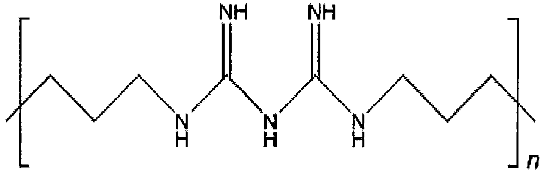

It is usually used as a heterodisperse mixture of PHMB polymer hydrochlorides of different lengths with an average molecular weight of approximately 3,000 Da, which can be represented by the general formula:

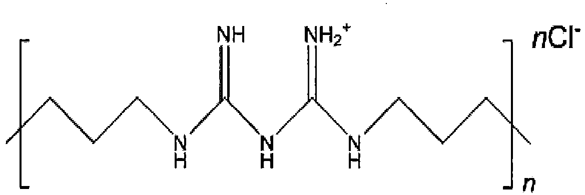

PHMB polymer hydrochloride is sold under various trade names such as Vantocil IB. (Vantocil and Vantocil IB are Trade Marks of Arch Chemicals, Inc.)

At low concentrations, polymeric biguanides of this general type are non-toxic and non-irritant.

Preferably the composition comprises a mixture a PHMB molecules having a molecular weight ranging from 500 to 20,000 Da and having preferably an average molecular weight of about 3,000 Da such as the biguanide polymer marketed under the Trade Name Vantocil IB.

The concentration by weight of each of the at least one biguanide polymer, the at least one further active agent and the at least one tertiary amine oxide is preferably lower than 5% w/w, more preferably lower than 3% w/w or most preferably lower than 1.5% w/w.

In the formulation/composition, typically the total amount of biguanide polymer present is in the range of 0.05 to 5, 3 or 1.5 % w/w, preferably it is in the range of 0.1 to 1 % w/w, more preferably it is in the range of 0.15 to 0.5 % w/w, most preferably it is in the range of 0.17 to 0.25 % w/w. Typically the total amount of tertiary amine oxide present is in the range of 0.05 to 5, 3 or 1.5 % w/w, preferably it is in the range of 0.1 to 1.3 % w/w, more preferably it is in the range of 0.5 to 1.2 % w/w, most preferably it is in the range of 0.7 to 1.1 % w/w. Typically, when quaternary ammonium salts are present in the formulation, the total amount of quaternary ammonium salts present is in the range of 0.1 to 5 % w/w, preferably it is in the range of 0.5 to 3 or 2.5 % w/w, more preferably it is in the range of 1 to 3 or 2.5 % w/w and most preferably it is in the range of 1.5 to 3 or 2.5 % w/w.

For formulations suitable for application to the skin, preferably, the total amount of quaternary ammonium salts is less than 2.5 % w/w.

The total concentration of the at least one biguanide polymer, the at least one further active agent and the at least one tertiary amine oxide is preferably lower than 10% w/w and more preferably lower than 7%, or 5% w/w.

For formulations suitable for application to the skin, such as hand sanitizer formulations, preferably, the total concentration of the at least one biguanide polymer, the at least one further active agent and the at least one tertiary amine oxide is preferably lower than 8 % w/w and more preferably lower than 6 %, or 4 % w/w.

The unexpectedly enhanced biocidal activity of the composition enables the use of a lower concentration of active agents (including biguanide polymer) and amine oxide. Thus, the composition of the present invention causes less skin and/or eye irritation, in use, and is less environmentally damaging to manufacture and to dispose of, than previously known compositions having a similar level of biocidal activity.

Preferably, the biocidal composition has a sporicidal activity of a log₁₀4, or log₁₀5, or greater reduction in viable spore (e.g. C. difficile spore) concentrations under standard test EN13704 conducted in dirty conditions, in 10 or fewer minutes, or more preferably 5 or fewer minutes.

Surprisingly, the biocidal composition achieves both an unusually high log reduction in viable spore concentrations (in both clean and dirty conditions), but the said concentration is reduced at an unusually short period of time. This is particularly surprising in relation to embodiments wherein each said constituent of the composition is, when taken alone, sporicidally inactive, and more particularly aqueous compositions wherein each said constituent of the composition is, when taken alone, sporicidally inactive.

The composition may further comprise at least one polyorganosiloxane (also known as silicone polymers).

Preferably, the composition further comprises a polyorganosiloxane or a mixture thereof. One or more, or all of the or each polyorganosiloxane may be a functionalised polyorganosiloxane, comprising polar functional groups or more preferably ionic, or cationic, functional groups. One or more, or all of the or each polyorganosiloxane may be functionalised along the length of the polyorganosiloxane. One or more, or all of the or each functionalised polyorganosiloxane may be a functionally terminated polyorganosiloxane (i.e. a polyorganosiloxane wherein polymer chains are terminated at one or both ends by a functional group, which may be a polar or, more preferably an ionic, or cationic, functional group).

Functionalised polyorganosiloxanes and in particular functionally terminated polyorganosiloxanes (such as cationic functionally terminated polyorganosiloxanes) interact strongly with surfaces (which are typically negatively charged) and compositions of the invention comprising such polyorganosiloxanes are particularly suitable for the treatment of materials and surfaces. Functionalised polyorganosiloxanes, and in particular functionally terminated polyorganosiloxanes (such as cationic functionally terminated polyorganosiloxanes) may similarly have strong interactions with other polar molecules in the composition, which may be polymeric biguanides and further active agents, and thereby retain such polar molecules to any material or surface to which the composition is applied and provide the material or surface with biocidal properties for a period of time (and in some cases an extended period of days, weeks or months).

Polyorganosiloxanes which can be used in the invention are of the type having the formula A:

Formula A:

T¹R¹R²SiO(R³R⁴SiO)ᵣ(R⁵T²SiO)ₛSiR⁶R⁷T³ wherein the symbols R¹, R², R³, R⁴, R⁵, R⁶ and R⁷, which are identical or different, represent a hydrogen, phenyl or a C₁ - C₆ alkyl radical, and the symbols T¹, T² and T³, which are identical or different, represent a hydrogen, phenyl, a C₁-C₆ alkyl radical, or an alkyl group of the form

-R⁸OR⁹X

wherein R⁸ and R⁹, which are identical or different, represent a C₁ to C₆ alkyl radical containing none, one or more secondary alcohol groups, and X represents a primary alcohol or a quaternary amine of the form

-(NR¹⁰R¹¹R¹²)⁺Z⁻

wherein R¹⁰, R¹¹, and R¹², which are identical or different, represent C₁-C₁₉ alkyl or alkene groups, either linear or branched chain, and Z represents an acetate anion, and
r is an average value ranging from 1 to 500;
s is an average value ranging from 1 to 10.

It is preferred to use a composition, wherein the polyorganosiloxane is a polymer of formula B:

Formula B: T(Me)₂SiO(SiMe₂0)ᵣ(SiMeTO)ₛSiMe₂T

wherein
r is an average value ranging from 1 to 100, preferably 80,
s is an average value ranging from 1 to 10, preferably 2, 15
T represents a group of the formula:

   -(CH₂)₃OCH₂CHOHCH₂X

   wherein X represents a primary alcohol, or an amine of the form:

   -NMe₂Y⁺Acetate⁻

   wherein Y represents a C₁₁-C₁₃ alkyl.

The polyorganosiloxane or the mixture of polyorganosiloxanes may be present in the composition in an amount of up to 10 % w/w, 0% or 0. 5 to 10% w/w. Preferably, the polyorganosiloxane or the mixture of polyorganosiloxanes is present in the composition in an amount of 0.2 to 1.5 or 5 % w/w. In some embodiments, the polyorganosiloxane or the mixture of polyorganosiloxanes is present in an amount of from 10% to 50% w/w and may be present in the amount of from 30% to 40% w/w. For example, the polyorganosiloxane or the mixture of polyorganosiloxanes may be present in an amount from 0% to 10% w/w in a composition comprising water as a diluent, the said composition being then applied to a surface (such as the surface of a fabric) and being fixed thereto by drying so as to remove all or a substantial part of the water, the resulting composition comprising an amount from 5 or 10 to 40 % w/w or 5 or 10% to 50% w/w of the polyorganosiloxane or the mixture of polyorganosiloxanes.

One or more of the one or more further active agents may be contained in a non volatile carrier. Accordingly, the composition may further comprise one or more non-volatile carriers. The or each non-volatile carrier may be water, a glycol, an ester derivative of a glycol or an ether derivative of a glycol. In particular, alkylene glycols and poly alkylene glycols such as diethylene glycol and polyethylene glycol may be used. Alternatively, the or each carrier may be an alcohol having from 3 to 30 carbon atoms, preferably at least 10 carbon atoms.

The composition may further comprise other ingredients, such as surfactants, chelating agents (such as aminocarboxylates, (ethylenediaminetetra-acetates, nitrilotriacetates, N,N-bis(carboxymethyl)-glutamates, citrates), alcohols (ethanol, isopropanol, glycols) detergency adjuvants (phosphates, silicates), dyes, and fragrances. In some embodiments, the composition comprises a surfactant which is a said tertiary amine oxide. Thus, advantageously, the composition may comprise an amine oxide (e.g. a long chain alkyl amine oxide) which may be a surfactant, which therefore both substantially enhances the activity of other active components of the composition (in comparison to compositions lacking the amine oxide(s) or comprising the amine oxide(s) but lacking the other active components of the compositions) and improves the dispersion of the compositions over a surface. In some embodiments (in particular in aqueous biocidal compositions according to the present invention), the composition is foaming or foamable, as a result of the surfactant properties of the or each said amine oxide.

The composition can comprise emollients such as coconut diethanolamide and fatty acid monoalkanolamide ethoxylate (where the fatty acid is coco fatty acid) for some applications, such as where it is applied to skin, for example. Typically, total emollients can be present in the formulation in an amount of up to 3 % w/w.

The composition may comprise a diluent, excipient or carrier, which, in some embodiments, is a non-volatile diluent, excipient or carrier. Such carrier is preferably water as a water-based composition is both safe and environmentally friendly. The composition is preferably an aqueous composition, i.e. wherein more than 70% or 80% or 90% of the composition by mass is water, in which the other constituents of the composition are dissolved, mixed or dispersed.

The composition is preferably alcohol free (by which we mean that the composition contains no, or only a small proportion - less than 1% w/w or in some cases approximately 0.6% w/w alcohol).

One or more of the constituents of the composition (e.g. the or each further active agent) may be contained in a non volatile carrier, such as an alcohol or a glycol. Accordingly, the composition may further comprise one or more non-volatile carriers, and in preferred embodiments, the said carriers may be present in only a low concentration of less than 1% w/w, or approximately 0.6% w/w, such that the composition is substantially free of the said carriers. In embodiments wherein one or more of the constituents are alcohols or other volatile organic compounds ("VOCs"), the composition is alcohol-free, or more generally VOC-free (by which we mean that the composition contains no, or only a small proportion - less than 1% w/w or in some cases approximately 0.6% w/w VOCs).

A textile may be impregnated with a composition as defined above. The textile may be woven or non-woven and may comprise synthetic and/or natural fibres. This permits a method of imparting sporicidal properties to a textile by impregnating said textile with a composition as defined above.

The impregnated textile may be wet, for example a surface wipe impregnated with an amount of the composition (e.g. soaked in an amount of aqueous composition, and suitable for use to disinfect a surface by wiping the said surface). In some embodiments the textile is dried. That is to say that the composition may be fixed to a surface by removing all or a substantial part of the diluent, excipient or carrier (e.g. by causing the diluent, excipient or carrier to evaporate by heating and/or evacuation).

The invention permits a method of disinfecting a surface, comprising applying an effective amount of a composition as defined above, to a surface. This method can be a method of imparting sporicidal activity to said surface, i.e. a method of reducing the number of viable spores on said surface or a method of killing spores on said surface. Preferably the spores include those of *Bacillus subtilis*, *Bacillus cereus and*/or *Clostridium Difficile.*

The surface may be inanimate and may be any of the surfaces typically found in a hospital or ambulances such as floors, walls, ceilings, surfaces of furniture, surfaces of medical instruments. The surface may be animate, such as human skin, for example, the skin on human hands.

In one embodiment, the composition of the invention is applied to the surface using a dry film fogging machine. The composition of the invention which does not include isothiazolines is particularly suitable for this application.

Preferably, the composition is applied to a surface in the form of a foam.

In embodiments where the composition used is biocidal, once a surface has been disinfected using the method of the current aspect of the invention, the surface may remain residually biocidally active. That is, the surface may remain biocidally active for a period of time after the composition has been applied. The surface may remain biocidally active after the non-volatile carrier of the composition has at least partially evaporated. The surface may remain biocidally active for at least 2 hours after the composition has been applied. The surface may remain biocidally active for at least 3 hours after the composition has been applied. The surface may remain biocidally active for at least 4 hours after the composition has been applied.

The provision of a method of disinfecting a surface where the surface remains biocidally active for up to 4 hours, for example, ensures that a surface remains clean and free of contaminents for a significant period after the surface has been disinfected, allowing the surface to be cleaned less frequently, using a smaller amount of biocidal composition, without compromising the well being of any patients coming into contact with the said surface, for example.

It is possible to use a pump dispenser containing an amount of a composition as defined above, which is preferably a foaming or foamable composition (i.e. such that the said composition is dispensable from the dispenser in the form of a foam).

Accordingly, the composition is used as a sporicide. The composition has been found to be particularly effective at killing spores, i.e. reducing the number of viable spores.

A method of enhancing the activity of a disinfectant composition can comprise the steps of providing a solution of at least one tertiary amine oxide and the said disinfectant composition. Preferably, the method is a method of enhancing the sporicidal activity of a disinfectant composition.

The disinfectant composition comprises at least one biguanide polymer as described above. The disinfectant composition comprises at least one further active agents as described above.

The method may be a method of providing a disinfectant composition with sporicidal activity. i.e. The disinfectant composition may show low or no activity towards spores, and may not meet the EN 13704 standard, in the absence of the or each said tertiary amine oxide, and a mixture of the disinfectant composition with an amount (typically less than 5% w/w and preferably less than 1.5% w/w) of tertiary amine oxide, may demonstrate sporicidal activity.

The method may comprise providing a solution of at least one tertiary amine oxide and the said disinfectant composition, to thereby provide a biocidal composition as described above.

In a further aspect, the invention extends to the use of a tertiary amine oxide to enhance the activity of a disinfectant composition. Preferably, the use is to enhance the sporicidal activity of a sporicidal composition.

In a further aspect, the invention extends to the provision of a biocidal composition as described in relation to the first aspect of the invention suitable to be used as a hand sanitizer, wherein the concentration by weight of each of the at least one biguanide polymer, the at least one further active agent, and the at least one tertiary amine oxide is lower than 3% w/w. Accordingly the present invention provides for the use of such composition as a hand sanitizer, i.e. for disinfecting skin. In said use the composition can be applied to the skin in the form of a foam, solution or cream.

By a disinfectant or biocidal composition, we mean a composition showing one or more or all of biocidal, anti-microbial, bactericidal, fungicidal, germicidal, yeasticidal, moldicidal, algicidal, virucidal or sporicidal activity.

Herein, the unit of concentration % w/w means percentage by weight. For example, if a composition comprises a gram of a first compound and 99 g of a second compound, the first compound has a concentration of 1% w/w and the second compound has a concentration of 99% w/w.

Herein, the unit of concentration ppm means parts per million, by mass. For example, if 100 g of a composition comprises a gram of a first compound, the first compound is present in a concentration of 10,000 ppm, and therefore a concentration of 1% w/w corresponds to a concentration of 10,000 ppm.

Further preferred and optional features of the further aspects correspond to preferred and optional features of the first aspect.

### Detailed Description of an Example Embodiment

Embodiments of the present invention will now be described by way of a non- limiting example only:
Formulations 1 and 2 were prepared having the following compositions:

### Formulation 1 - without tertiary amine oxide

A biocidal composition was made by mixing the following source raw materials in water: Acticide BAC 50M (comprising a 70:30 ratio of, respectively, C12 and C14 alkyldimethylbenzylammonium chloride at a total concentration of 50% w/w, in water), Acticide DDQ 50 (comprising 50% w/w di-n-decyldimethylammonium chloride and 20% w/w propan-2-ol co-solvent, in water), Surfac CM 50A (comprising at least 90% Fatty Acid MonoAlkanolamide Ethoxylate), Surfac CDE (comprising at least 90% Coconut Diethanolamide) and Vantocil IB (comprising 20% w/w polyhexamethylene biguanide hydrochloride, in water). The chemical formulation was as follows in ppm by mass:
Acticide DDQ 50 and Acticide BAC 50M were purchased from Thor Specialities (UK) Ltd. of Northwich, UK. Surfac AO30, Surfac CM50A and Surfac CDE were purchased from Surfachem of Leeds, UK. Vantocil IB was purchased from Arch Chemicals, Inc. of Norwalk, Connecticut, USA.

| **Chemical** | **Concentration** | **Source Raw Material** |
|---|---|---|
| Di-n-decyl dimethylammonium chloride | 11,000ppm | Acticide DDQ 50 |
| Alkyldimethylbenzylammonium chloride 70% C12 and 30% C14 | 11,000ppm | Acticide BAC 50M |
| Fatty Acid MonoAlkanolamide Ethoxylate | 10,000ppm | Surfac CM50A |
| Propan-2-ol | 4,400ppm | Acticide DDQ 50 |
| Coconut Diethanolamide | 5,000ppm | Surfac CDE |
| Citric Acid | 300ppm | |
| Polyhexamethylene biguanide | 2,400ppm | Vantocil IB |
| Water | Balance | |

### Formulation 2 - with tertiary amine oxide

A biocidal composition was prepared having a similar composition to Formulation 1, but further comprising a mixture of alkyldimethylamine oxides by adding Surfac AO30, the balance of the composition being made up by water such that the concentrations of remaining compounds were the same as Formulation 1.

The chemical formulation was as follows in ppm by mass:

| **Chemical** | **Concentration** | **Source Raw Material** |
|---|---|---|
| Di-n-decyl dimethylammonium chloride | 11,000ppm | Acticide DDQ 50 |
| Alkyldimethylbenzylammonium chloride 70% C12 and 30% C14 | 11,000ppm | Acticide BAC 50M |
| C10-C16 AlkylDimethylAmine Oxide | 10,000ppm | Surfac AO30 |
| Fatty Acid MonoAlkanolamide Ethoxylate | 10,000ppm | Surfac CM50A |
| Propan-2-ol | 4,400ppm | Acticide DDQ 50 |
| Coconut Diethanolamide | 5,000ppm | Surfac CDE |
| Citric Acid | 300ppm | |
| Polyhexamethylene biguanide | 2,400ppm | Vantocil IB |
| Water | Balance | |

### Formulation 3 - hand sanitizer formulation

A biocidal composition was prepared having a similar composition to Formulation 2, wherein the formulation has been diluted by 20%.

The chemical formulation was as follows in ppm by mass:

| **Chemical** | **Concentration** | **Source Raw Material** |
|---|---|---|
| Di-n-decyl dimethylammonium chloride | 8,800ppm | Acticide DDQ 50 |
| Alkyldimethylbenzylammonium chloride 70% C12 and 30% C14 | 8,800ppm | Acticide BAC 50M |
| C10-C16 AlkylDimethylAmine Oxide | 8,000ppm | Surfac AO30 |
| Fatty Acid MonoAlkanolamide Ethoxylate | 8,000ppm | Surfac CM50A |
| Propan-2-ol | 3,250ppm | Acticide DDQ 50 |
| Coconut Diethanolamide | 4,000ppm | Surfac CDE |
| Citric Acid | 240ppm | |
| Polyhexamethylene biguanide | 1,920ppm | Vantocil IB |
| Water | Balance | |

### Sporicidal Activity

Biocidal activity of Formulations 1, 2 and 3 were tested according to the protocol of the standard suspension test EN 13704 for bacteria *Clostridium difficile,* except that anaerobic conditions were used to culture the bacteria

EN 13704 is a European standard procedure to test the efficacy of sporicidal disinfectants. The procedure specifies that the formulation to be tested be used against *Bacillus subtilis, Bacillus cereus* or *Clostridium sporogenes.* To pass the EN 13074 test, a formulation must have a log reduction of at least 3 (0.1% of organisms surviving) in 60 minutes.

The above formulations were tested against *C*. *difficile* for a measurement more directly relevant for medical applications.

1 ml of a *C.difficile* stock suspension was mixed with 1 ml of soil, to which 8 ml of the formulation to be tested was added and maintained at 20 °C. After the determined contact time (specified in the standard procedure as 60 minutes, but for fast acting biocides, such as the biocides of the current invention, shorter contact times are necessary, such as 1 minute or 5 minutes, for example) 1 ml of solution was removed and added to 9 ml of recovery or neutraliser solution (such as Tryptone Soya Broth containing Tween 80 10%, Lecithin 3%, Sodium thiosulphate 0.5%, Cystine 0.1% and Hystidine 0.1%). The resulting solution was then plated out to detect surviving organisms. For each sample removed the spores within the sample were counted and the reduction in viable counts was calculated.

The resulting log reduction counts, given in the table below, correspond to the common logarithm of the number of surviving organisms subtracted from the common logarithm of the original number of organisms, such that a log reduction of 1 corresponds to 10% of organisms surviving.

| | Formulation 1 | Formulation 2 | Formulation 3 |
|---|---|---|---|
| Log Reduction after 5 minutes | 3.1 | 6.7 | 5.5 |

The log reduction for Formulation 2 is 3.6 greater than that for Formulation 1. Thus, the addition of the tertiary amine oxide to the formulation produces an increase in the log reduction of 3.6, equating to a difference of approximately 1000 times greater efficacy. Given that tertiary amine oxides are not known to have any significant sporicidal activity, this result is very surprising.

Thus, the combination of polymeric biguanide, the amine oxide and any or all of the remaining active agents in Formulation 2 results in an unexpected sporicidal activity, which may be regarded as a synergistic effect between the polymeric biguanide, the amine oxide and the further active agents. Furthermore, the further active agents each result in an unexpected enhancement of the sporicidal activity of a composition comprising a polymeric biguanide and an amine oxide, which may also be regarded as a synergistic effect between each further active agent, the polymeric biguanide and the amine oxide and/or each other further active agent, in the presence of the polymeric biguanide.

In addition, the log reduction obtained for Formulation 3, which is suitable for use as a hand sanitizer, is the highest known to the applicant for a biocidal composition suitable for use as a hand sanitizer. Accordingly, Formulation 3 provides a highly effective hand sanitizer that has a sufficiently low concentration of active agents to be safe for use on the skin and yet provide a high log reduction of c.difficile spores.

### Residual Sporicidal Activity

A culture of *C.difficile* spores was applied to the surface of 150 mm square tiles and allowed to dry (0.5 ml containing 0.3% Bovine albumin). Sporicidal Formulation 2 was applied to the tiles in the form of a foam using a pump dispenser, where each pump of disinfectant corresponds to approximately 1.4 ml of liquid. After application the tile was wiped with a wet surface wipe, and after five minutes the tile was wiped, surviving organisms were recovered from the tile and textile wipe, and counted.

A log reduction of at least 6.7 was observed, equating to the elimination of every spore in the sample.

After a period of four hours, the surface was inoculated with a further culture of spores (the surface was "challenged" with spores). After a further period of five minutes a swab was taken from the surface and the log reduction observed for spores is shown below:

| Spore type | Log reduction observed |
|---|---|
| *C. difficile* | 3.8 |

As can be seen, even four hours after a surface has been disinfected using Formulation 2, a log reduction of 3.8 is observed in *C.difficile* spores. That is, the surface is sporicidally active for at least four hours after it has been disinfected. This provides a major benefit allowing the surface to be cleaned less frequently whilst not compromising the well being of those in contact with the said surface.

The above protocol was followed after inoculating a previously disinfected surface with *E. coli* bacteria and a log reduction of 5.5 was observed.

Similar results have been obtained for Formulation 3, resulting in a hand sanitizer that provides long lasting protection against contaminants such as c.difficile spores.

Further variations and modifications may be made within the scope of the invention herein disclosed.

## Claims

1. A non-therapeutic method of killing spores on a surface which comprises applying an effective amount of a composition to said surface, wherein said composition comprises at least one biguanide polymer, at least one further active agent and at least one tertiary amine oxide, wherein the at least one further active agent comprises a quaternary ammonium salt.

2. A non-therapeutic method according to claim 1, wherein the at least one said tertiary amine oxide comprises a tertiary alkyl amine oxide.

3. A non-therapeutic method according to claim 1 or claim 2, wherein the composition comprises a di-n-decyldimethylammonium salt and/or an alkylbenzyldimethyl ammonium salt.

4. A non-therapeutic method according to any one preceding claim, wherein the composition has a sporicidal activity of a log₁₀5 or greater reduction in viable C. difficile spore concentrations using the protocol of the standard test EN13704 conducted in dirty conditions, in 5 or fewer minutes.

5. A non-therapeutic method according to any one preceding claim, wherein the composition further comprises at least one polyorganosiloxane.

6. A non-therapeutic method according to any one of the preceding claims, wherein the composition is a foaming or foamable composition.

7. A non-therapeutic method according to any one of the preceding claims, wherein the method comprises wiping said surface with a textile impregnated with said composition.

8. A non-therapeutic method according to any one of claims 1 to 5, wherein the method comprises using a dry film fogging machine.

9. Composition for use as a sporicidal hand sanitiser, wherein said composition comprises at least one biguanide polymer, at least one further active agent and at least one tertiary amine oxide, wherein the at least one further active agent comprises a quaternary ammonium salt and wherein the concentration by weight of each of the at least one biguanide polymer, the at least one further active agent, and the at least one tertiary amine oxide is lower than 3% w/w.

10. Composition for use according to claim 9 wherein the at least one tertiary amine oxide is a tertiary alkyl amine oxide.

11. Composition for use according to claim 10 wherein the composition comprises a di-n-decyldimethylammonium salt and/or an alkylbenzyldimethyl ammonium salt.

12. Composition for use according to any one of claims 9 to 11 wherein the composition is a foaming or foamable composition.

13. Composition for use according to any one of claims 9 to 12, wherein the composition has a sporicidal activity of a log₁₀5 or greater reduction in viable C. difficile spore concentrations using the protocol of the standard test EN 13704 conducted in dirty conditions, in 5 or fewer minutes.

14. Use of a tertiary amine oxide to enhance the sporicidal activity of a disinfectant composition comprising at least one biguanide polymer, at least one further active agent and at least one tertiary amine oxide, wherein the at least one further active agent comprises a quaternary ammonium salt.

15. Use according to claim 14 wherein the at least one tertiary amine oxide is a tertiary alkyl amine oxide.

## Patentansprüche

1. Nicht-therapeutisches Verfahren zum Abtöten von Sporen auf einer Oberfläche, welches das Auftragen einer wirksamen Menge einer Zusammensetzung auf die Oberfläche umfasst, wobei die Zusammensetzung wenigstens ein Biguanidpolymer, wenigstens einen weiteren Wirkstoff und wenigstens ein tertiäres Aminoxid enthält, wobei der wenigstens eine weitere Wirkstoff ein quartäres Ammoniumsalz enthält.

2. Nicht-therapeutisches Verfahren nach Anspruch 1, wobei das wenigstens eine tertiäre Aminoxid ein tertiäres Alkylaminoxid enthält.

3. Nicht-therapeutisches Verfahren nach Anspruch 1 oder 2, wobei die Zusammensetzung ein Di-n-Decyldimethylammoniumsalz und/oder ein Alkylbenzyldimethylammoniumsalz enthält.

4. Nicht-therapeutisches Verfahren nach einem der vorhergehenden Ansprüche, wobei die Zusammensetzung eine sporizide Wirkung von einer log₁₀ (5)-fachen oder größeren Reduzierung lebensfähiger *C.-difficile*-Sporenkonzentrationen aufweist, unter Verwendung des Protokolls des Standardtests EN13704, durchgeführt unter unreinen Bedingungen, innerhalb von 5 min oder weniger.

5. Nicht-therapeutisches Verfahren nach einem der vorhergehenden Ansprüche, wobei die Zusammensetzung weiter wenigstens ein Polyorganosiloxan enthält.

6. Nicht-therapeutisches Verfahren nach einem der vorhergehenden Ansprüche, wobei es sich bei der Zusammensetzung um eine schaumige oder aufschäumbare Zusammensetzung handelt.

7. Nicht-therapeutisches Verfahren nach einem der vorhergehenden Ansprüche, wobei das Verfahren das Abwischen der Oberfläche mit einem Textil umfasst, das mit der Zusammensetzung getränkt ist.

8. Nicht-therapeutisches Verfahren nach einem der Ansprüche 1-5, wobei das Verfahren die Verwendung eines Trockenfilm-Nebelgeräts umfasst.

9. Zusammensetzung zur Verwendung als sporizides Handdesinfektionsmittel, wobei die Zusammensetzung wenigstens ein Biguanidpolymer, wenigstens einen weiteren Wirkstoff und wenigstens ein tertiäres Aminoxid enthält, wobei der wenigstens eine weitere Wirkstoff ein quartäres Ammoniumsalz enthält und wobei die Konzentration, bezogen auf das Gewicht von jedem von dem wenigstens einen Biguanidpolymer, dem wenigstens einen weiteren Wirkstoff, und dem wenigstens einen tertiären Aminoxid, geringer ist als 3 Gew.-%.

10. Zusammensetzung zur Verwendung nach Anspruch 9, wobei es sich bei dem wenigstens einen tertiären Aminoxid um ein tertiäres Alkylaminoxid handelt.

11. Zusammensetzung zur Verwendung nach Anspruch 10, wobei die Zusammensetzung ein Di-n-Decyldimethylammoniumsalz und/oder ein Alkylbenzyldimethylammoniumsalz enthält.

12. Zusammensetzung zur Verwendung nach einem der Ansprüche 9 bis 11, wobei es sich bei der Zusammensetzung um eine schaumige oder aufschäumbare Zusammensetzung handelt.

13. Zusammensetzung zur Verwendung nach einem der Ansprüche 9 bis 12, wobei die Zusammensetzung eine sporizide Wirkung von einer log₁₀ (5)-fachen oder größeren Reduzierung lebensfähiger *C.-difficile-*Sporenkonzentrationen aufweist, unter Verwendung des Protokolls des Standardtests EN13704, durchgeführt unter unreinen Bedingungen, innerhalb von 5 min oder weniger.

14. Verwendung eines tertiären Aminoxids, um die sporizide Wirkung einer Desinfektionsmittelzusammensetzung zu verstärken, die wenigstens ein Biguanidpolymer, wenigstens einen weiteren Wirkstoff und wenigstens ein tertiäres Aminoxid enthält, wobei der wenigstens eine weitere Wirkstoff ein quartäres Ammoniumsalz enthält.

15. Verwendung nach Anspruch 14, wobei es sich bei dem wenigstens einen tertiären Aminoxid um ein tertiäres Alkylaminoxid handelt.

## Revendications

1. Méthode non thérapeutique de destruction de spores sur une surface, comprenant l'application d'une quantité efficace d'une composition à ladite surface, où ladite composition comprend au moins un polymère de biguanide, au moins un autre agent actif et au moins un oxyde d'amine tertiaire, où le au moins un autre agent actif comprend un sel d'ammonium quaternaire.

2. Méthode non thérapeutique selon la revendication 1, où ledit au moins un oxyde d'amine tertiaire comprend un oxyde d'alkylamine tertiaire.

3. Méthode non thérapeutique selon la revendication 1 ou la revendication 2, où la composition comprend un sel de di-n-décyldiméthylammonium et/ou un sel d'alkyl-benzyldiméthylammonium.

4. Méthode non thérapeutique selon l'une quelconque des revendications précédentes, où la composition possède une activité sporicide d'une réduction de log₁₀5 ou plus des concentrations en spores de *C*. *difficile* viables en utilisant le protocole de l'essai standard EN13704 effectué en conditions sales, en 5 minutes ou moins.

5. Méthode non thérapeutique selon l'une quelconque des revendications précédentes, où la composition comprend en outre au moins un polyorganosiloxane.

6. Méthode non thérapeutique selon l'une quelconque des revendications précédentes, où la composition est une composition moussante ou pouvant mousser.

7. Méthode non thérapeutique selon l'une quelconque des revendications précédentes, où la méthode comprend l'essuyage de ladite surface par un textile imprégné par ladite composition.

8. Méthode non thérapeutique selon l'une quelconque des revendications 1 à 5, où la méthode comprend l'utilisation d'un brumisateur à film sec.

9. Composition pour une utilisation comme désinfectant sporicide pour les mains, où ladite composition comprend au moins un polymère de biguanide, au moins un autre agent actif et au moins un oxyde d'amine tertiaire, où le au moins un autre agent actif comprend un sel d'ammonium quaternaire, et où la concentration en poids de chacun parmi le au moins un polymère de biguanide, le au moins un autre agent actif et le au moins un oxyde d'amine tertiaire est inférieure à 3% p/p.

10. Composition pour une utilisation selon la revendication 9, où le au moins un oxyde d'amine tertiaire est un oxyde d'alkylamine tertiaire.

11. Composition pour une utilisation selon la revendication 10, où la composition comprend un sel de di-n-décyldiméthylammonium et/ou un sel d'alkylbenzyl-diméthylammonium.

12. Composition pour une utilisation selon l'une quelconque des revendications 9 à 11, où la composition est une composition moussante ou pouvant mousser.

13. Composition pour une utilisation selon l'une quelconque des revendications 9 à 12, où la composition possède une activité sporicide d'une réduction de log₁₀5 ou plus des concentrations en spores de *C*. *difficile* viables en utilisant le protocole de l'essai standard EN13704 effectué en conditions sales, en 5 minutes ou moins.

14. Utilisation d'un oxyde d'amine tertiaire afin d'améliorer l'activité sporicide d'une composition de désinfectant comprenant au moins un polymère de biguanide, au moins un autre agent actif et au moins un oxyde d'amine tertiaire, où le au moins un autre agent actif comprend un sel d'ammonium quaternaire.

15. Utilisation selon la revendication 14, où le au moins un oxyde d'amine tertiaire est un oxyde d'alkylamine tertiaire.
